# EUROPEAN PATENT SPECIFICATION

(11) **EP 4 527 314 B1**
(45) Date of publication and mention of the grant of the patent: **08.04.2026**
(21) Application number: 24201189.8
(22) Date of filing: 18.09.2024
(51) Int. Cl.: A61B 8/08, A61B 8/14, A61B 8/00

(54) **ULTRASOUND DIAGNOSTIC APPARATUS AND CONTROL METHOD OF ULTRASOUND DIAGNOSTIC APPARATUS**
ULTRASCHALLDIAGNOSEVORRICHTUNG UND STEUERUNGSVERFAHREN FÜR DIE ULTRASCHALLDIAGNOSEVORRICHTUNG
APPAREIL DE DIAGNOSTIC À ULTRASONS ET PROCÉDÉ DE COMMANDE D'APPAREIL DE DIAGNOSTIC À ULTRASONS

(30) Priority: 21.09.2023 JP 2023155558
(43) Date of publication of application: 26.03.2025
(73) Proprietor: FUJIFILM Corporation, Tokyo 106-8620 (JP)
(72) Inventor: MATSUMOTO, Tsuyoshi, Kanagawa (JP)
(74) Representative: Dehns Germany Partnerschaft mbB

(56) References cited:
- US-A1- 2021 093 303
- US-A1- 2022 160 332
- US-A1- 2022 175 345
- US-A1- 2023 000 461

## Description

### BACKGROUND OF THE INVENTION

### 1. Field of the Invention

The present invention relates to an ultrasound diagnostic apparatus and a control method of the ultrasound diagnostic apparatus that acquire a three-dimensional structure of a subject.

### 2. Description of the Related Art

In the related art, an examination is performed by capturing an ultrasound image representing a tomogram of a subject using a so-called ultrasound diagnostic apparatus. In such an examination, a user such as a doctor usually captures the ultrasound image while changing a posture of a so-called ultrasound probe and moving the ultrasound probe in a state where the ultrasound probe is in contact with a body surface of the subject.

In this case, the user usually sequentially captures ultrasound images while determining a captured part of the subject by checking the captured ultrasound image, but a user having a low skill level in the examination using the ultrasound diagnostic apparatus may have difficulty in determining which part of the subject is imaged even by checking the ultrasound image. Therefore, for example, as disclosed in JP2011-104137A, a technique of estimating an imaging point of the subject and displaying the imaging point to the user has been developed. In JP2011-104137A, three-dimensional shape data of a general subject is prepared, three-dimensional image data of the subject is generated from a plurality of frames of ultrasound images captured during the examination, and the imaging point of the subject is estimated by comparing the three-dimensional shape data with the three-dimensional image data.

US 2021/093303 discloses a medical image diagnostic apparatus, ultrasonic diagnostic apparatus, medical imaging system, and imaging control method. US 2022/160332 discloses an apparatus and method for automatic ultrasound segmentation for visualization and measurement.

### SUMMARY OF THE INVENTION

However, in general, since the shape and arrangement of the part inside the subject vary greatly depending on the subject, in the technique of JP2011-104137A which uses three-dimensional shape data of a general subject in order to estimate the imaging point of the subject, the imaging point of the subject may not be correctly estimated, and the user may not accurately ascertain the imaging point of the subject.

The present invention has been made to solve such a problem in the related art, and an object thereof is to provide an ultrasound diagnostic apparatus and a control method of the ultrasound diagnostic apparatus which allow a user to be able to easily ascertain an imaging point of a subject regardless of a skill level.

Therefore, the user can easily ascertain the imaging point of the subject regardless of the skill level.

### BRIEF DESCRIPTION OF THE DRAWINGS

Fig. 1 is a block diagram illustrating a configuration of an ultrasound diagnostic apparatus according to a first embodiment of the present invention.
Fig. 2 is a block diagram illustrating a configuration of a transmission and reception circuit in the first embodiment of the present invention.
Fig. 3 is a block diagram illustrating a configuration of an image generation unit in the first embodiment of the present invention.
Fig. 4 is a flowchart illustrating an operation of the ultrasound diagnostic apparatus according to the first embodiment of the present invention.
Fig. 5 is a block diagram illustrating a configuration of an ultrasound diagnostic apparatus according to a second embodiment of the present invention.
Fig. 6 is a block diagram illustrating a configuration of an ultrasound diagnostic apparatus according to a third embodiment of the invention.
Fig. 7 is a diagram illustrating an example of a three-dimensional schema image in the third embodiment of the present invention.
Fig. 8 is a block diagram illustrating a configuration of an ultrasound diagnostic apparatus according to a fourth embodiment of the present invention.
Fig. 9 is a block diagram illustrating a configuration of an ultrasound diagnostic apparatus according to a fifth embodiment of the present invention.
Fig. 10 is a block diagram illustrating a configuration of an ultrasound diagnostic apparatus according to a sixth embodiment of the present invention.

### DESCRIPTION OF THE PREFERRED EMBODIMENTS

The invention is set out in the appended set of claims.

Hereinafter, embodiments of the invention will be described with reference to the accompanying drawings.

The description of configuration requirements described below is given on the basis of a representative embodiment of the invention, but the invention is not limited to such an embodiment.

Note that, in the present specification, a numerical range represented using "to" means a range including numerical values before and after "to" as a lower limit value and an upper limit value.

In the present specification, the terms "same" and "identical" include an error range generally allowed in the technical field.

### First Embodiment

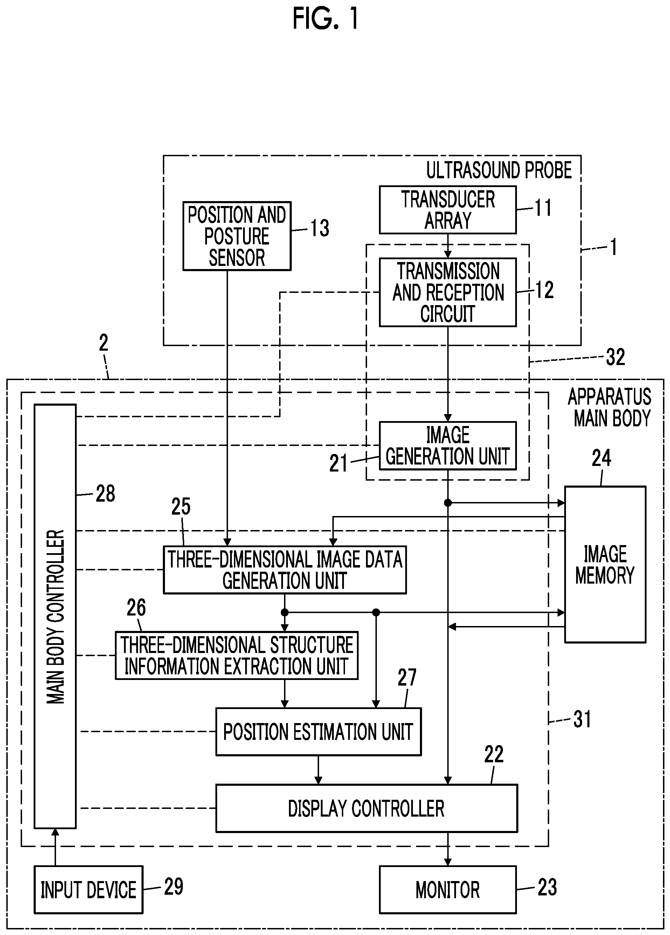

Fig. 1 illustrates a configuration of an ultrasound diagnostic apparatus according to a first embodiment of the invention. The ultrasound diagnostic apparatus comprises an ultrasound probe 1, and an apparatus main body 2 connected to the ultrasound probe 1. The ultrasound probe 1 and the apparatus main body 2 are connected to each other by so-called wired communication or so-called wireless communication.

The ultrasound probe 1 includes a transducer array 11. A transmission and reception circuit 12 is connected to the transducer array 11. In addition, the ultrasound probe 1 includes a position and posture sensor 13. The position and posture sensor 13 may be built into the ultrasound probe 1, or may be attached to a housing of the ultrasound probe 1. In addition, for example, in a case where a sensor device that measures the ultrasound probe 1 from the outside, such as a so-called optical sensor, is used as the position and posture sensor 13, the position and posture sensor 13 may be disposed outside the ultrasound probe 1.

The apparatus main body 2 includes an image generation unit 21 connected to the transmission and reception circuit 12 of the ultrasound probe 1. A display controller 22 and a monitor 23 are sequentially connected to the image generation unit 21. An image memory 24 is connected to the image generation unit 21. The display controller 22 is connected to the image memory 24. A three-dimensional image data generation unit 25 is connected to the position and posture sensor 13 and the image memory 24. A three-dimensional structure information extraction unit 26 is connected to the three-dimensional image data generation unit 25. In addition, a position estimation unit 27 is connected to the three-dimensional image data generation unit 25 and the three-dimensional structure information extraction unit 26. The position estimation unit 27 is connected to the display controller 22.

In addition, a main body controller 28 is connected to the transmission and reception circuit 12, the image generation unit 21, the display controller 22, the image memory 24, the three-dimensional image data generation unit 25, the three-dimensional structure information extraction unit 26, and the position estimation unit 27. An input device 29 is connected to the main body controller 28. In addition, a processor 31 for the apparatus main body 2 is configured by the image generation unit 21, the display controller 22, the three-dimensional image data generation unit 25, the three-dimensional structure information extraction unit 26, the position estimation unit 27, and the main body controller 28. In addition, the transmission and reception circuit 12 and the image generation unit 21 constitute an image acquisition unit 32.

The transducer array 11 of the ultrasound probe 1 has a plurality of ultrasonic transducers arranged in a one-dimensional or two-dimensional manner. According to a drive signal supplied from the transmission and reception circuit 12, each of the ultrasonic transducers transmits an ultrasonic wave and receives an ultrasound echo from the subject to output a signal based on the ultrasound echo. For example, each ultrasonic transducer is configured by forming electrodes at both ends of a piezoelectric body consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

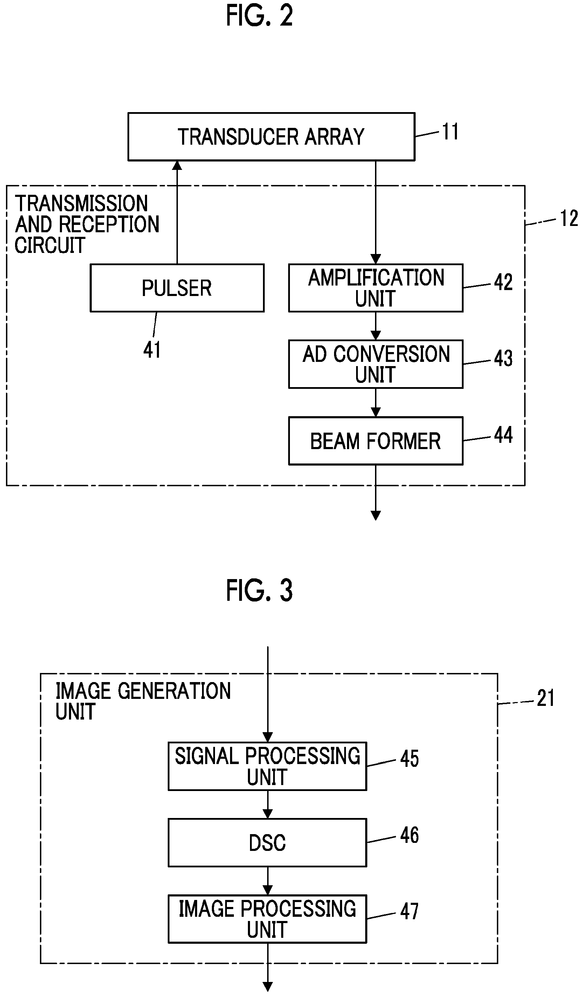

The transmission and reception circuit 12 causes the transducer array 11 to transmit the ultrasound wave and generates a sound ray signal on the basis of a reception signal acquired by the transducer array 11, under the control of the main body controller 28. As illustrated in Fig. 2, the transmission and reception circuit 12 includes a pulser 41 connected to the transducer array 11, and an amplification unit 42, an analog-to-digital (AD) conversion unit 43, and a beam former 44 that are sequentially connected in series to the transducer array 11.

The pulser 41 includes, for example, a plurality of pulse generators, and the pulser 41 adjusts the amount of delay of each drive signal so that ultrasonic waves transmitted from the plurality of ultrasonic transducers of the transducer array 11 form an ultrasound beam on the basis of a transmission delay pattern selected according to the control signal from the main body controller 28, and supplies the obtained signals to the plurality of ultrasonic transducers. In this way, in a case where a pulsed or continuous wave-like voltage is applied to the electrodes of the ultrasound transducer of the transducer array 11, the piezoelectric body expands and contracts to generate a pulsed or continuous wave-like ultrasound wave from each of the ultrasound transducers, whereby an ultrasound beam is formed from the combined wave of these ultrasound waves.

The transmitted ultrasound beam is reflected by a target, for example, a site of the subject, and propagates toward the transducer array 11 of the ultrasound probe 1. The ultrasound echo propagating toward the transducer array 11 in this way is received by each of the ultrasound transducers constituting the transducer array 11. In this case, each of the ultrasound transducers constituting the transducer array 11 receives the propagating ultrasound echo to expand and contract to generate a reception signal, which is an electrical signal, and outputs these reception signals to the amplification unit 42.

The amplification unit 42 amplifies the signal input from each of the ultrasound transducers constituting the transducer array 11, and transmits the amplified signal to the AD conversion unit 43. The AD conversion unit 43 converts the signal transmitted from the amplification unit 42 into digital reception data. The beam former 44 performs so-called reception focus processing by applying and adding a delay to each reception data received from the AD conversion unit 43. By this reception focus processing, each reception data converted by the AD conversion unit 43 is phase-added, and a sound ray signal in which the focus of the ultrasound echo is narrowed down is acquired.

As illustrated in Fig. 3, the image generation unit 21 has a configuration in which a signal processing unit 45, a digital scan converter (DSC) 46, and an image processing unit 47 are sequentially connected in series.

The signal processing unit 45 generates a B-mode image signal, which is tomographic image information regarding tissues inside the subject, by performing, on the sound ray signal received from the transmission and reception circuit 12, correction of the attenuation due to the distance according to the depth of the reflection position of the ultrasound wave using a sound velocity value set by the main body controller 28 and then performing envelope detection processing.

The DSC 46 converts (raster-converts) the B-mode image signal generated by the signal processing unit 45 into an image signal in accordance with a normal television signal scanning method.

The image processing unit 47 performs various kinds of necessary image processing such as gradation processing on the B-mode image signal input from the DSC 46, and then transmits the B-mode image signal to the display controller 22 and the image memory 24. Hereinafter, the B-mode image signal that has been subjected to image processing by the image processing unit 47 is referred to as an ultrasound image.

The image memory 24 is a memory that stores the ultrasound image acquired by the image acquisition unit 32 and the three-dimensional image data, which will be described later, generated by the three-dimensional image data generation unit 25. Here, as the image memory 24, for example, recording media such as a flash memory, a hard disk drive (HDD), a solid state drive (SSD), a flexible disk (FD), a magneto-optical disk (MO disk), a magnetic tape (MT), a random access memory (RAM), a compact disc (CD), a digital versatile disc (DVD), a secure digital card (SD card), or a universal serial bus memory (USB memory) can be used.

The position and posture sensor 13 of the ultrasound probe 1 is a sensor device that acquires position and posture information of the ultrasound probe 1. Here, in general, in a case where a user performs an examination of a subject using an ultrasound diagnostic apparatus, the user often performs the examination while changing a posture, that is, an angle of the ultrasound probe 1 and moving the position of the ultrasound probe 1 in a state where the ultrasound probe 1 is in contact with the body surface of the subject. The position and posture information of the ultrasound probe 1 acquired by the position and posture sensor 13 includes information regarding the posture and position of the ultrasound probe 1. For example, the position and posture sensor 13 can include at least one of a so-called inertial sensor, a magnetic sensor, or an optical sensor. The inertial sensor can include, for example, at least one of a so-called acceleration sensor or a gyro sensor.

The three-dimensional image data generation unit 25 generates three-dimensional ultrasound image data of the subject on the basis of the position and posture information of the ultrasound probe 1 acquired by the position and posture sensor 13 and the ultrasound image acquired by the image acquisition unit 32. In this case, the three-dimensional image data generation unit 25 can generate the three-dimensional image data by, for example, associating the ultrasound images of the plurality of frames acquired by the image acquisition unit 32 with the position and posture information of the ultrasound probe 1 acquired by the position and posture sensor 13 at the timing at which each of the ultrasound images of the plurality of frames is acquired, and arranging the ultrasound images of the plurality of frames in accordance with the corresponding position and posture information.

The three-dimensional image data generation unit 25 can associate, for example, the ultrasound image acquired by the image acquisition unit 32 with the position and posture information that is input from the position and posture sensor 13 within a certain period of time including immediately before a timing of moment at which the ultrasound image is input to the three-dimensional image data generation unit 25 via the image memory 24. In addition, in a case where a time stamp indicating the acquisition time point is added to the position and posture information in the position and posture sensor 13 and a time stamp indicating the acquisition time point is added to the ultrasound image in the image acquisition unit 32, for example, the three-dimensional image data generation unit 25 can associate the position and posture information with the ultrasound image with each other in a case where a difference between the time stamp added to the position and posture information and the time stamp added to the ultrasound image is within a certain range.

The three-dimensional image data generated by the three-dimensional image data generation unit 25 in this way is transmitted to the image memory 24, the three-dimensional structure information extraction unit 26, and the position estimation unit 27.

The three-dimensional structure information extraction unit 26 extracts three-dimensional structure information regarding a three-dimensional structure included in the three-dimensional ultrasound image data, from the three-dimensional ultrasound image data generated by the three-dimensional image data generation unit 25. The three-dimensional structure information extraction unit 26 can extract, for example, a running pattern of a blood vessel of the subject included in the three-dimensional ultrasound image data, as the three-dimensional structure information. Since the running pattern of the blood vessel, particularly the vein, has a relatively small difference between the subjects, it is useful for ascertaining the three-dimensional structure in the subject. In addition, the three-dimensional structure information extraction unit 26 can also extract, for example, a shape pattern, a size, and the like of a structure present around the blood vessel, such as a diaphragm or an intestinal tract, as the three-dimensional structure information in addition to the running pattern of the blood vessel. In addition, the three-dimensional structure information extraction unit 26 can also extract a brightness pattern of the three-dimensional structure as the three-dimensional structure information.

The position estimation unit 27 has a position estimation model that is trained in a position of a three-dimensional structure in three-dimensional image data obtained by imaging the three-dimensional structure of the subject, and estimates a position of a region occupied by the three-dimensional ultrasound image data of the subject by the position estimation model on the basis of the three-dimensional structure information extracted by the three-dimensional structure information extraction unit 26.

The position estimation model is a so-called machine learning model, and as the position estimation model, a so-called deep learning model, a so-called support vector machine (SVM), a so-called decision tree model, or the like can be used. The position estimation model is trained in advance in a relationship between a position of a three-dimensional structure in a large amount of three-dimensional image data and a position of a region occupied by three-dimensional ultrasound image data of a subject, and outputs the position of the region occupied by the three-dimensional ultrasound image data of the subject in response to input of the three-dimensional structure information. The position estimation model can learn, as the position of the three-dimensional structure in the three-dimensional image data, a position of a running pattern of a blood vessel in a subject and a position of a shape pattern of a structure present around the blood vessel, such as a diaphragm or an intestinal tract. In addition, the position estimation model can also learn the size of the shape pattern of the structure present around the blood vessel.

Note that the position of the region occupied by the three-dimensional ultrasound image data of the subject refers to a position of a region in a subject corresponding to the three-dimensional ultrasound image data.

In addition, as the three-dimensional image data to be learned by the position estimation model, three-dimensional ultrasound image data, three-dimensional image data formed by a so-called computed tomography (CT) image, or three-dimensional image data formed by a so-called magnetic resonance imaging (MRI) image can be used.

The position of the region occupied by the three-dimensional ultrasound image data of the subject estimated by the position estimation unit 27 in this way is displayed on the monitor 23 via the display controller 22. The position estimation unit 27 can, for example, display the position of the region occupied by the three-dimensional ultrasound image data of the subject by superimposing the position on a three-dimensional human body model representing the entire subject or a part of the subject such as the abdomen.

Meanwhile, the user usually sequentially captures ultrasound images while determining a captured part of the subject by checking the captured ultrasound image, but the user having a low skill level in the examination using the ultrasound diagnostic apparatus may have difficulty in determining which part of the subject is imaged even by checking the ultrasound image. Since the position estimation unit 27 in the first embodiment can accurately estimate the position of the region occupied by the three-dimensional ultrasound image data of the subject by using the position estimation model, the user can easily ascertain the imaging point in the subject by checking the estimated position of the region occupied by the three-dimensional ultrasound image data.

The display controller 22 performs predetermined processing on the ultrasound image transmitted from the image acquisition unit 32 and the image memory 24, the three-dimensional image data of the subject generated by the three-dimensional image data generation unit 25, and the position of the region occupied by the three-dimensional ultrasound image data of the subject estimated by the position estimation unit 27 to display the resultant on the monitor 23, under the control of the main body controller 28.

The monitor 23 displays the ultrasound image, the three-dimensional image data, and the position of the region occupied by the three-dimensional ultrasound image data of the subject under the control of the display controller 22, and has, for example, a display device such as a liquid crystal display (LCD) and an organic electroluminescence display (organic EL display).

The main body controller 28 controls each unit of the apparatus main body 2 and the transmission and reception circuit 12 of the ultrasound probe 1 on the basis of a control program and the like stored in advance.

The input device 29 is for a user to perform an input operation, and is configured by, for example, a device such as a keyboard, a mouse, a trackball, a touchpad, and a touch sensor superimposed on the monitor 23.

Note that the processor 31 having the image generation unit 21, the display controller 22, the three-dimensional image data generation unit 25, the three-dimensional structure information extraction unit 26, the position estimation unit 27, and the main body controller 28 is configured by a central processing unit (CPU) and a control program for causing the CPU to execute various kinds of processing, but the processor 31 may be configured by using a field programmable gate array (FPGA), a digital signal processor (DSP), an application specific integrated circuit (ASIC), a graphics processing unit (GPU), or other integrated circuits (IC) or may be configured by a combination thereof.

In addition, the image generation unit 21, the display controller 22, the three-dimensional image data generation unit 25, the three-dimensional structure information extraction unit 26, the position estimation unit 27, and the main body controller 28 of the processor 31 can be partially or wholly integrated into one CPU or the like.

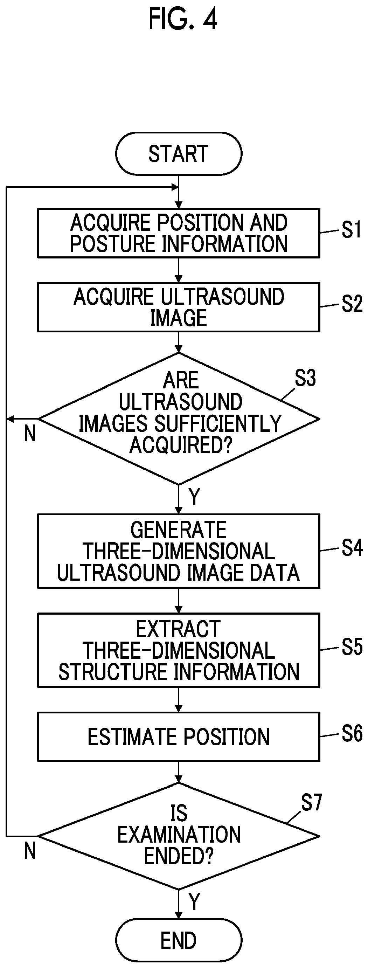

Next, the operation of the ultrasound diagnostic apparatus according to the first embodiment will be described with reference to the flowchart illustrated in Fig. 4. Hereinafter, it is assumed that the user brings the ultrasound probe 1 into contact with the body surface of the subject.

In step S1, the position and posture sensor 13 acquires the position and posture information of the ultrasound probe 1. The position and posture information of the ultrasound probe 1 acquired in step S1 is transmitted to the three-dimensional image data generation unit 25.

In step S2, the image acquisition unit 32 acquires the ultrasound image of the subject. In this case, under the control of the main body controller 28, the transmission and reception of ultrasonic waves from the plurality of transducers of the transducer array 11 are started according to the drive signal from the pulser 41 of the transmission and reception circuit 12 of the ultrasound probe 1, the ultrasound echo from the subject is received by the plurality of transducers of the transducer array 11, and the reception signal as the analog signal is output to the amplification unit 42 to be amplified, and then is subjected to the AD conversion by the AD conversion unit 43 to acquire the reception data.

The reception focus processing is performed on the reception data by the beam former 44, and the sound ray signal generated by the reception focusing processing is transmitted to the image generation unit 21 of the apparatus main body 2. An ultrasound image representing tomographic image information of the subject is generated by the image generation unit 21. In this case, the signal processing unit 45 of the image generation unit 21 performs the correction of the attenuation according to the depth of the reflection position of the ultrasonic wave and the envelope detection processing on the sound ray signal, the DSC 46 performs the conversion into the image signal according to a normal television signal scanning method, and the image processing unit 47 performs various kinds of necessary image processing such as gradation processing. The ultrasound image acquired in step S2 in this way is displayed on the monitor 23 via the display controller 22 and is transmitted to the three-dimensional image data generation unit 25 via the image memory 24.

The position and posture information of the ultrasound probe 1 acquired in step S1 and the ultrasound image acquired in step S2 are associated with each other by the three-dimensional image data generation unit 25. The three-dimensional image data generation unit 25 can associate, for example, the ultrasound image acquired in step S1 with the position and posture information input within a certain period of time including immediately before a timing of moment at which the ultrasound image is input to the three-dimensional image data generation unit 25.

In step S3, the main body controller 28 determines whether or not the ultrasound images are sufficiently acquired in generating the three-dimensional ultrasound image data. The main body controller 28 can determine whether or not the ultrasound images are sufficiently acquired, for example, by determining whether or not a predetermined period of time has elapsed from the start of step S1. For example, the main body controller 28 can determine that the ultrasound images can be sufficiently acquired in a case where a predetermined period of time has elapsed after the start of step S1, and determine that the ultrasound images cannot be sufficiently acquired in a case where the predetermined period of time has not yet elapsed.

In addition, for example, the main body controller 28 can determine whether or not the ultrasound images are sufficiently acquired by determining whether or not the movement of the ultrasound probe 1 is stopped after the ultrasound probe 1 is moved by the user by referring to the position and posture information acquired in step S1. The main body controller 28 can determine, for example, that the ultrasound images can be sufficiently acquired in a case where the movement of the ultrasound probe 1 is stopped after the ultrasound probe 1 is moved by the user, and determine that the ultrasound images cannot be sufficiently acquired in a case where the movement of the ultrasound probe 1 is continued.

In step S3, in a case where it is determined that the ultrasound images to be used for generating the three-dimensional ultrasound image data are not sufficiently acquired, the processing returns to step S1, the position and posture information of the ultrasound probe 1 is newly acquired by the position and posture sensor 13, the ultrasound images are newly acquired by the image acquisition unit 32 in subsequent step S2, and it is determined in step S3 whether or not the ultrasound images are sufficiently acquired. In this way, the processing of steps S1 to S3 is repeated until it is determined in step S3 that the ultrasound images are sufficiently acquired.

In a case where it is determined in step S3 that the ultrasound images to be used for generating the three-dimensional ultrasound image data are sufficiently acquired, the processing proceeds to step S4. In step S4, the three-dimensional image data generation unit 25 generates the three-dimensional ultrasound image data of the subject on the basis of the plurality of pieces of position and posture information of the ultrasound probe 1 obtained by repeating step S1 and the ultrasound images of the plurality of frames obtained by repeating step S2. In this case, the three-dimensional image data generation unit 25 can generate the three-dimensional ultrasound image data by, for example, arranging the ultrasound images of the plurality of frames obtained by repeating step S2 in accordance with the position and posture information of the ultrasound probe 1 associated with each ultrasound image.

In step S5, the three-dimensional structure information extraction unit 26 extracts the three-dimensional structure information regarding the three-dimensional structure included in the three-dimensional ultrasound image data, from the three-dimensional ultrasound image data generated in step S4. The three-dimensional structure information extraction unit 26 can extract, for example, a running pattern of a blood vessel of the subject included in the three-dimensional ultrasound image data, and a shape pattern and a size of a structure present around the blood vessel, as the three-dimensional structure information.

In step S6, the position estimation unit 27 estimates the position of the region occupied by the three-dimensional ultrasound image data of the subject by the position estimation model on the basis of the three-dimensional structure information extracted in step S5. The position estimation model is trained in advance by a machine learning method in a relationship between a position of a three-dimensional structure in a large amount of three-dimensional ultrasound image data and a position of a region occupied by the three-dimensional ultrasound image data of the subject, and outputs the position of the region occupied by the three-dimensional ultrasound image data of the subject in response to input of the three-dimensional structure information.

The position of the region occupied by the three-dimensional ultrasound image data of the subject estimated by the position estimation unit 27 in this way is displayed on the monitor 23. The position estimation unit 27 can, for example, display the position of the region occupied by the three-dimensional ultrasound image data of the subject by superimposing the position on the three-dimensional human body model representing the entire subject or a part of the subject such as the abdomen.

The structure inside the subject has individual differences depending on the subject, but the position estimation unit 27 estimates the position of the region occupied by the three-dimensional ultrasound image data of the subject by the position estimation model. Therefore, even in a case where the structure inside the subject is different from, for example, an ideal structure, the position of the region occupied by the three-dimensional ultrasound image data of the subject can be accurately estimated. Therefore, the user can easily ascertain the imaging point of the subject regardless of the user's skill level by checking the position of the region occupied by the three-dimensional ultrasound image data of the subject displayed on the monitor 23.

As a result, the user can easily ascertain, for example, whether or not a site to be examined inside the subject is imaged comprehensively. In addition, since the position estimation unit 27 can accurately estimate the position of the region occupied by the three-dimensional ultrasound image data of the subject, the user can comprehensively image the site to be examined in the subject. Therefore, for example, it is easy to find a local lesion, such as a tumor, present around the blood vessel. In addition, the user having a low skill level can easily improve the skill level by performing the imaging while checking the position of the region occupied by the three-dimensional ultrasound image data of the subject displayed on the monitor 23, and it is possible for a skilled person to save the time and effort for checking the imaging result.

Finally, in step S7, the main body controller 28 determines whether or not to end the examination of the subject. The main body controller 28 can determine, for example, to end the examination in a case where the user determines that the examination for the subject is sufficiently performed, and inputs an instruction to end the examination via the input device 29, and the main body controller 28 can determine to continue the examination in a case where the user does not particularly input an instruction to end the examination via the input device 29.

In a case where it is determined to continue the examination in step S7, the processing returns to step S1. Thereafter, the processing of steps S1 to S7 is performed again. In a case where it is determined to end the examination in step S7, the operation of the ultrasound diagnostic apparatus according to the flowchart of Fig. 4 is completed.

As described above, with the ultrasound diagnostic apparatus according to the first embodiment, the three-dimensional image data generation unit 25 generates the three-dimensional ultrasound image data of the subject on the basis of the position and posture information of the ultrasound probe 1 acquired by the position and posture sensor 13 and the ultrasound image acquired by the image acquisition unit 32, the three-dimensional structure information extraction unit 26 extracts the three-dimensional structure information regarding the three-dimensional structure included in the three-dimensional ultrasound image data, from the three-dimensional ultrasound image data generated by the three-dimensional image data generation unit 25, and the position estimation unit 27 has the position estimation model that is trained in the position of the three-dimensional structure in the three-dimensional image data obtained by imaging the three-dimensional structure, and estimates the position of the region occupied by the three-dimensional ultrasound image data of the subject by the position estimation model on the basis of the three-dimensional structure information extracted by the three-dimensional structure information extraction unit 26. Therefore, the position of the region occupied by the three-dimensional ultrasound image data of the subject is accurately estimated and the user can easily ascertain the imaging point of the subject regardless of the skill level.

Note that the description has been made in which the transmission and reception circuit 12 is included in the ultrasound probe 1, but the transmission and reception circuit 12 may be included in the apparatus main body 2.

In addition, the description has been made in which the image generation unit 21 is included in the apparatus main body 2, but the image generation unit 21 may be included in the ultrasound probe 1.

In addition, the apparatus main body 2 may be a so-called stationary type, a portable type that is easy to carry, or a so-called handheld type configured by a smartphone or tablet computer. As described above, the type of equipment constituting the apparatus main body 2 is not particularly limited.

In addition, step S2 is performed after step S1 in the flowchart of Fig. 4, but step S1 may be performed after step S2, or step S1 and step S2 may be simultaneously performed.

In addition, the ultrasound diagnostic apparatus may include, for example, a coverage determination unit (not illustrated) that determines whether or not a region as an observation target is comprehensively imaged. The coverage determination unit is connected to, for example, the display controller 22, the position estimation unit 27, and the main body controller 28. The coverage determination unit can determine whether or not the region as the observation target is comprehensively imaged, for example, by determining whether or not a three-dimensional region of the observation target inside the subject, which is input by the user via the input device 29 and is set by the main body controller 28, is filled, without a gap, with a region corresponding to the three-dimensional ultrasound image data of which the position is estimated by the position estimation unit 27.

In this case, for example, a step of determining whether or not the imaging of the observation target is comprehensively performed by the coverage determination unit may be provided instead of step S7 in the flowchart of Fig. 4. For example, the main body controller 28 causes each unit of the ultrasound diagnostic apparatus to perform the processing of steps S1 to S6 until the coverage determination unit determines that the observation target is comprehensively imaged, and in a case where the coverage determination unit determines that the observation target is comprehensively imaged, the main body controller 28 can complete the operation of the ultrasound diagnostic apparatus. In this way, by determining whether or not the observation target is comprehensively imaged by the ultrasound diagnostic apparatus, the user can reliably perform the comprehensive imaging of the observation target.

### Second Embodiment

Due to various factors in acquiring the ultrasound images of the plurality of frames, a deviation may occur in the position and posture information of the ultrasound probe 1 corresponding to the acquired ultrasound images of the plurality of frames, and the surface of the three-dimensional structure in the three-dimensional ultrasound image data generated by the three-dimensional image data generation unit 25 may have irregularities that do not originally exist. Therefore, the ultrasound diagnostic apparatus can reconstruct the three-dimensional ultrasound image data such that the three-dimensional structure in the three-dimensional ultrasound image data has a smooth shape.

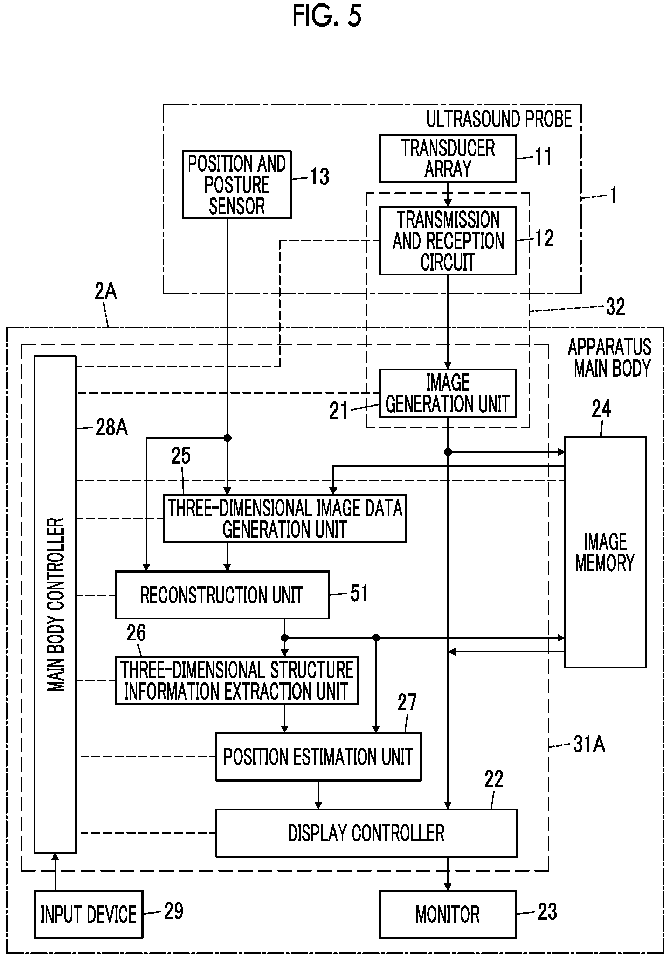

Fig. 5 is a block diagram illustrating a configuration of an ultrasound diagnostic apparatus according to a second embodiment. The ultrasound diagnostic apparatus of the present embodiment is obtained by including an apparatus main body 2A instead of the apparatus main body 2 in the ultrasound diagnostic apparatus of the first embodiment illustrated in Fig. 1. The apparatus main body 2A is obtained by further providing a reconstruction unit 51 to the apparatus main body 2 in the first embodiment, and including a main body controller 28A instead of the main body controller 28.

In the apparatus main body 2A, the reconstruction unit 51 is connected to the position and posture sensor 13 and the three-dimensional image data generation unit 25. The reconstruction unit 51 is connected to the image memory 24, the three-dimensional structure information extraction unit 26, the position estimation unit 27, and the main body controller 28A. **In** addition, a processor 31A for the apparatus main body 2A is configured by the image generation unit 21, the display controller 22, the three-dimensional image data generation unit 25, the three-dimensional structure information extraction unit 26, the position estimation unit 27, the main body controller 28A, and the reconstruction unit 51.

The reconstruction unit 51 extracts a two-dimensional tomographic image from the three-dimensional ultrasound image data generated by the three-dimensional image data generation unit 25, and reconstructs the three-dimensional ultrasound image data of the subject on the basis of the extracted tomographic image and the position and posture information of the ultrasound probe 1 acquired by the position and posture sensor 13. The reconstruction unit 51 can extract the region of the two-dimensional tomographic image of the structure included in the three-dimensional ultrasound image data by, for example, a method of pixel value analysis such as binarization or multivaluation of pixels in the three-dimensional ultrasound image data, a so-called template matching method, a so-called graph cut method, or a method using a deep learning model. The reconstruction unit 51 can smooth the surface of the three-dimensional structure in the three-dimensional ultrasound image data by further performing processing of smoothing the contour of the structure in the plurality of two-dimensional tomographic images, processing of performing interpolation between the plurality of two-dimensional tomographic images, and the like.

The three-dimensional structure included in the three-dimensional ultrasound image data can be made close to the shape of the actual three-dimensional structure in the subject by reconstructing the three-dimensional ultrasound image data by the reconstruction unit 51.

The three-dimensional structure information extraction unit 26 extracts the three-dimensional structure information from the three-dimensional ultrasound image data reconstructed by the reconstruction unit 51.

The position estimation unit 27 estimates the position of the region occupied by the three-dimensional ultrasound image data of the subject, from the three-dimensional structure information extracted by the three-dimensional structure information extraction unit 26 by using the position estimation model.

In this way, since the position of the region occupied by the three-dimensional ultrasound image data of the subject is estimated on the basis of the three-dimensional structure information extracted from the three-dimensional ultrasound image data reconstructed by the reconstruction unit 51, the estimation accuracy of the position of the region occupied by the three-dimensional ultrasound image data of the subject can be improved as compared with a case where the position of the region occupied by the three-dimensional ultrasound image data of the subject is estimated on the basis of the three-dimensional structure information extracted as it is from the three-dimensional ultrasound image data generated by the three-dimensional image data generation unit 25.

### Third Embodiment

The ultrasound diagnostic apparatus can also display the region already imaged in the subject on the monitor 23 in an emphasized manner such that the user can clearly ascertain the region already imaged in the subject.

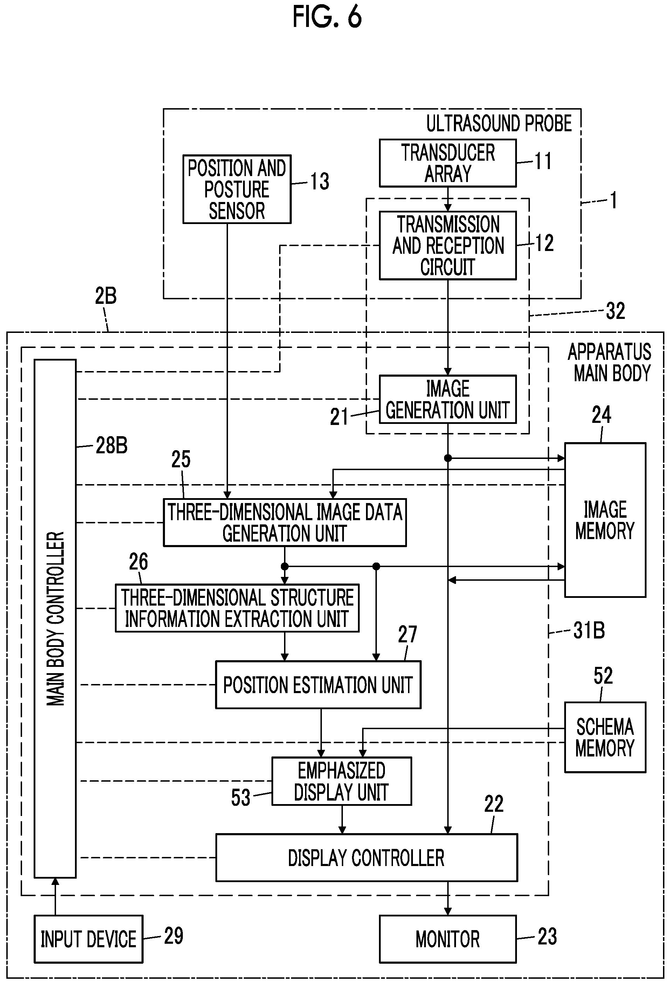

Fig. 6 is a block diagram illustrating a configuration of an ultrasound diagnostic apparatus according to a third embodiment. The ultrasound diagnostic apparatus of the third embodiment is obtained by including an apparatus main body 2B instead of the apparatus main body 2 in the ultrasound diagnostic apparatus of the first embodiment illustrated in Fig. 1. The apparatus main body 2B is obtained by further providing a schema memory 52 and an emphasized display unit 53 to the apparatus main body 2 in the first embodiment, and including a main body controller 28B instead of the main body controller 28.

In the apparatus main body 2B, the schema memory 52 is connected to the main body controller 28B. In addition, the emphasized display unit 53 is connected to the position estimation unit 27 and the schema memory 52. The emphasized display unit 53 is connected to the display controller 22 and the main body controller 28B. In addition, a processor 31B for the apparatus main body 2B is configured by the image generation unit 21, the display controller 22, the three-dimensional image data generation unit 25, the three-dimensional structure information extraction unit 26, the position estimation unit 27, the main body controller 28B, and the emphasized display unit 53.

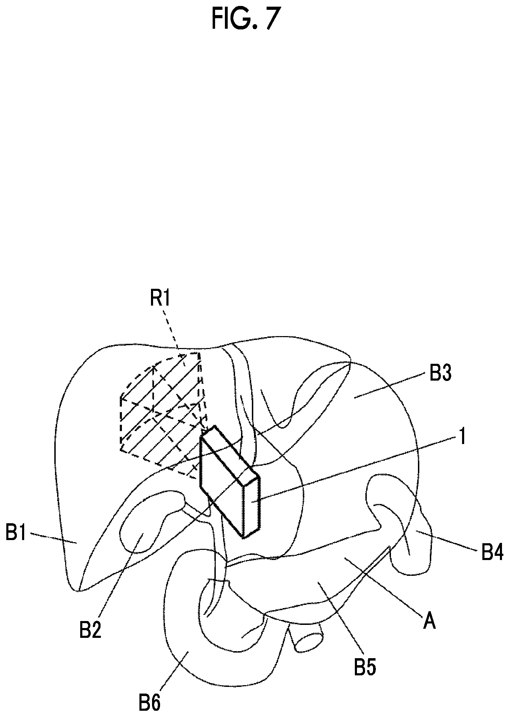

As illustrated in Fig. 7, for example, the schema memory 52 is a memory that stores a plurality of three-dimensional schema images A, which are three-dimensional schematic diagrams that mimic the structure of an organ or the like in the subject, in advance. Here, Fig. 7 illustrates an example of the three-dimensional schema image A including a liver B1, a gallbladder B2, a stomach B3, a spleen B4, a pancreas B5, and a duodenum B6. As the schema memory 52, for example, recording media such as a flash memory, an HDD, an SSD, an FD, an MO disk, an MT, a RAM, a CD, a DVD, an SD card, or a USB memory can be used.

As illustrated in Fig. 7, for example, the emphasized display unit 53 displays, in an emphasized manner, an occupied region R1 of the three-dimensional ultrasound image data of which the position is estimated by the position estimation unit 27, in the three-dimensional schema image A on the monitor 23. In the example of Fig. 7, the schematic diagram of the ultrasound probe 1 is illustrated to indicate that the occupied region R1 corresponds to the ultrasound images of the plurality of frames generated while the ultrasound probe 1 is inclined in a certain angle range, but the schematic diagram of the ultrasound probe 1 may not be illustrated.

The emphasized display unit 53 can read out and display, for example, the three-dimensional schema image A designated by the user via the input device 29 among the plurality of three-dimensional schema images A stored in the schema memory 52, on the monitor 23. In addition, the emphasized display unit 53 can display the occupied region R1 in an emphasized manner by giving a color different from the surrounding color to the occupied region R1 of the three-dimensional ultrasound image data of which the position is estimated by the position estimation unit 27, by making the occupied region R1 blink, or the like.

The user can clearly and easily ascertain the site in the subject, of which the ultrasound image has already been captured, by checking the occupied region R1 displayed in an emphasized manner on the monitor 23. As a result, the user can reliably and comprehensively image the necessary examination site.

Note that the example has been described in which the three-dimensional schema image A designated by the user via the input device 29 is read out from the plurality of three-dimensional schema images A stored in the schema memory 52, but, for example, the emphasized display unit 53 can automatically select the three-dimensional schema image A including the position of the occupied region R1 estimated by the position estimation unit 27, from the schema memory 52 and can display the three-dimensional schema image A on the monitor 23.

### Fourth Embodiment

There may be a case where the position of the occupied region R1 cannot be estimated by the position estimation unit 27 in a case where the ultrasound image acquired by the image acquisition unit 32 is unclear due to some reason or the like. In this case, the ultrasound diagnostic apparatus can synthesize the three-dimensional ultrasound image data of which the position cannot be estimated with the occupied region R1 that is adjacent to the three-dimensional ultrasound image data and of which the position has already been estimated.

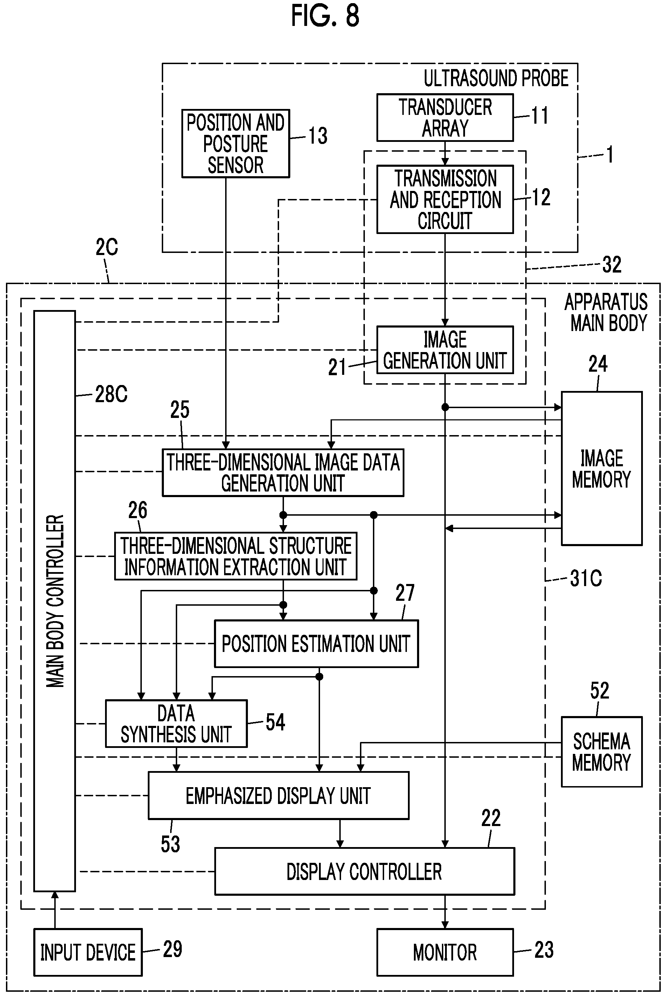

Fig. 8 is a block diagram illustrating a configuration of an ultrasound diagnostic apparatus according to a fourth embodiment. The ultrasound diagnostic apparatus of the fourth embodiment is obtained by further providing the schema memory 52, the emphasized display unit 53, and a data synthesis unit 54 to the ultrasound diagnostic apparatus of the first embodiment illustrated in Fig. 1, and including a main body controller 28C instead of the main body controller 28. Here, the schema memory 52 and the emphasized display unit 53 in the fourth embodiment are the same as the schema memory 52 and the emphasized display unit 53 in the third embodiment.

In an apparatus main body 2C, the data synthesis unit 54 is connected to the three-dimensional image data generation unit 25, the three-dimensional structure information extraction unit 26, and the position estimation unit 27. The data synthesis unit 54 is connected to the emphasized display unit 53 and the main body controller 28C. In addition, a processor 31C for the apparatus main body 2C is configured by the image generation unit 21, the display controller 22, the three-dimensional image data generation unit 25, the three-dimensional structure information extraction unit 26, the position estimation unit 27, the main body controller 28C, the emphasized display unit 53, and the data synthesis unit 54.

The data synthesis unit 54 sequentially synthesizes the three-dimensional ultrasound image data generated by the three-dimensional image data generation unit 25 based on the three-dimensional structure included in the three-dimensional ultrasound image data in which the position of the occupied region R1 is estimated by the position estimation unit 27.

Here, there may be a case where the position of the occupied region R1 cannot be estimated by the position estimation unit 27 in a case where the ultrasound image acquired by the image acquisition unit 32 is unclear due to some reason or the like. In this case, for example, the data synthesis unit 54 synthesizes the three-dimensional ultrasound image data of which the position cannot be estimated by the position estimation unit 27, with the three-dimensional structure included in the three-dimensional ultrasound image data that is adjacent to the three-dimensional ultrasound image data and in which the position of the occupied region R1 has already been estimated, with reference to the position and posture information of the ultrasound probe 1 acquired by the position and posture sensor 13.

The data synthesis unit 54 further synthesizes the three-dimensional ultrasound image data of the position adjacent to the three-dimensional ultrasound image data, with the synthesized three-dimensional ultrasound image data. In this manner, the data synthesis unit 54 can synthesize the three-dimensional ultrasound image data sequentially.

The emphasized display unit 53 displays, in an emphasized manner, the occupied region R1 of the three-dimensional ultrasound image data of which the position is estimated by the position estimation unit 27 and an occupied region R2 of the three-dimensional ultrasound image data synthesized by the data synthesis unit 54, in the three-dimensional schema image A.

Therefore, even in a case where the position estimation unit 27 cannot specify the position of the occupied region R1 for some reason, the user can comprehensively image the region to be examined while easily ascertaining the site in the subject of which the ultrasound image has already been captured, by checking the occupied regions R1 and R2 displayed in an emphasized manner on the three-dimensional schema image A.

### Fifth Embodiment

In a case where the position estimation unit 27 cannot estimate the position of the occupied region R1 of the three-dimensional ultrasound image data due to some reason, the ultrasound diagnostic apparatus may notify the user to return the ultrasound probe 1 to the posture and the position of the ultrasound probe 1 in a case where the three-dimensional ultrasound image data of which the position of the occupied region R1 has already been estimated is acquired.

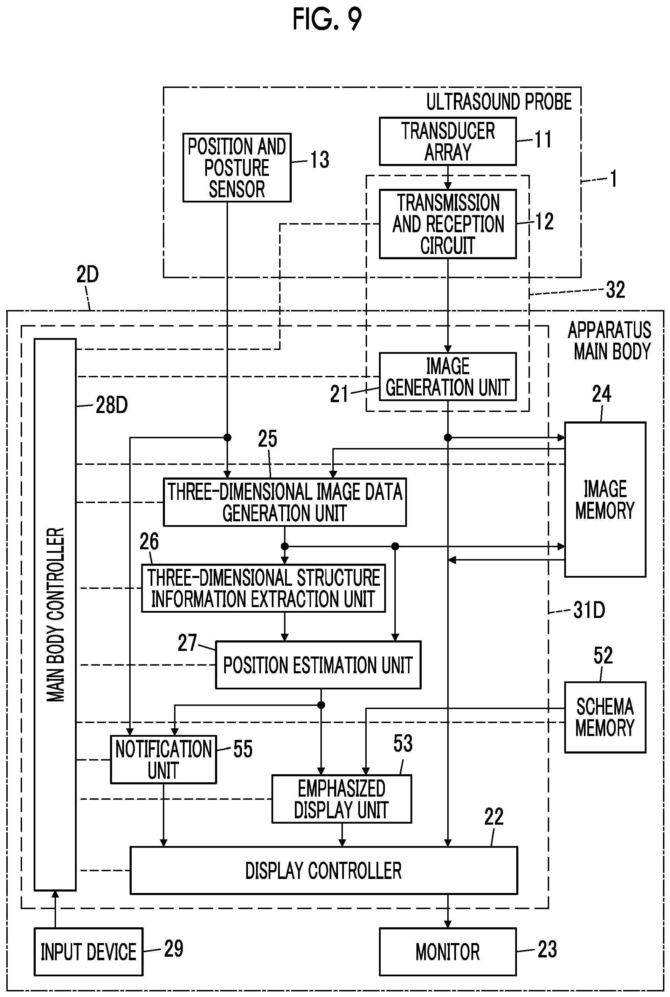

Fig. 9 is a block diagram illustrating a configuration of an ultrasound diagnostic apparatus according to a fifth embodiment. The ultrasound diagnostic apparatus of the fifth embodiment is obtained by including an apparatus main body 2D instead of the apparatus main body 2 in the ultrasound diagnostic apparatus of the first embodiment illustrated in Fig. 1. The apparatus main body 2D is obtained by further providing the schema memory 52, the emphasized display unit 53, and a notification unit 55 to the apparatus main body 2 in the first embodiment, and including a main body controller 28D instead of the main body controller 28. Here, the schema memory 52 and the emphasized display unit 53 in the fifth embodiment are the same as the schema memory 52 and the emphasized display unit 53 in the third and fourth embodiments.

In the apparatus main body 2D, the notification unit 55 is connected to the position and posture sensor 13 and the position estimation unit 27. The notification unit 55 is connected to the display controller 22 and the main body controller 28D. A processor 31D for the apparatus main body 2D is configured by the image generation unit 21, the display controller 22, the three-dimensional image data generation unit 25, the three-dimensional structure information extraction unit 26, the position estimation unit 27, the main body controller 28D, the emphasized display unit 53, and the notification unit 55.

In a case where the position estimation unit 27 cannot estimate the position of the occupied region R1 of the three-dimensional ultrasound image data, the notification unit 55 prompts the user to move the ultrasound probe 1 such that the ultrasound probe 1 is at the position and the posture of the ultrasound probe 1 in a case of acquiring the three-dimensional ultrasound image data of which the position of the occupied region R1 has already been estimated by the position estimation unit 27, on the basis of the position and posture information of the ultrasound probe 1 acquired by the position and posture sensor 13.

The notification unit 55 can acquire, for example, the last position and posture of the ultrasound probe 1 in a case of acquiring the three-dimensional ultrasound image data, with reference to the position and posture information in a case where the three-dimensional ultrasound image data of which the position of the occupied region R1 has already been estimated by the position estimation unit 27 is acquired. For example, the notification unit 55 can prompt the user to move the ultrasound probe 1 by displaying the position and the posture of the ultrasound probe 1 acquired as described above, on the monitor 23 in a superimposed manner on a schematic diagram (not illustrated) of the subject as viewed from the outside. In this case, the notification unit 55 can display, for example, a message such as "please move the ultrasound probe to the position and the posture of the ultrasound probe illustrated in the schematic diagram" on the monitor 23.

In addition, the notification unit 55 can also notify the user of a specific movement direction and the posture of the ultrasound probe 1 by displaying, on the monitor 23, a message such as "please move the ultrasound probe in the ×× direction by ×× cm and incline the ultrasound probe toward the body surface side by ×× degrees" on the basis of a difference obtained by calculating the difference between the last position and posture of the ultrasound probe 1 in a case of acquiring the three-dimensional ultrasound image data of which the position of the occupied region R1 has already been estimated by the position estimation unit 27 and the current position and posture of the ultrasound probe 1.

The user can return the ultrasound probe 1 to the position and the posture of the ultrasound probe 1 in a case of acquiring the three-dimensional ultrasound image data of which the position of the occupied region R1 has already been estimated by the position estimation unit 27, by checking the notification content by the notification unit 55, and acquire again the three-dimensional ultrasound image data of the position of the occupied region R1 that cannot be estimated by the position estimation unit 27, regardless of the skill level. As a result, the position of the occupied region R1 estimated by the position estimation unit 27 can be reliably estimated, and the occupied region R1 can be accurately displayed by the emphasized display unit 53. The user can more reliably and comprehensively image the necessary region in the subject while checking the occupied region R1 displayed in an emphasized manner on the monitor 23 as described above.

Note that the notification unit 55 can specify a region that is not displayed in an emphasized manner by the emphasized display unit 53 in the three-dimensional schema image A displayed on the monitor 23, and notify the user of the specified region. In this case, for example, the notification unit 55 can notify the user of the region that is not displayed in an emphasized manner by the emphasized display unit 53, by displaying the position of the ultrasound probe 1 on the body surface of the subject corresponding to the region that is not displayed in an emphasized manner in the three-dimensional schema image A and the posture of the ultrasound probe 1 at the position, on the monitor 23 in a superimposed manner on the schematic diagram (not illustrated) of the subject as viewed from the outside.

In addition, the notification unit 55 can notify the user of the region that is not displayed in an emphasized manner by the emphasized display unit 53, by estimating the position and the posture of the ultrasound probe 1 for imaging the region that is not displayed in an emphasized manner by the emphasized display unit 53 on the basis of the plurality of pieces of position and posture information already acquired by the position and posture sensor 13, and displaying a direction and a distance in which the current ultrasound probe 1 is to be moved and an angle at which the current ultrasound probe 1 is to be inclined, on the monitor 23 by using a message or the like, respectively.

By notifying the user of the region that is not displayed in an emphasized manner by the emphasized display unit 53 by the notification unit 55 in this way, the user can easily, reliably, and comprehensively image the region in the subject, which is to be examined.

In addition, the ultrasound diagnostic apparatus according to the fifth embodiment may include, for example, the data synthesis unit 54 as in the ultrasound diagnostic apparatus according to the fourth embodiment. Here, in a case where the position estimation unit 27 cannot estimate the position of the occupied region R1 and the data synthesis unit 54 synthesizes the three-dimensional ultrasound image data sequentially, a slight positional deviation occurring in a case of synthesizing the three-dimensional ultrasound image data may be accumulated, and thus the three-dimensional ultrasound image data may not be synthesized at the correct position.

Therefore, in a case where the three-dimensional ultrasound image data cannot be synthesized at the correct position, the notification unit 55 can prompt the user to move the ultrasound probe 1 such that the ultrasound probe 1 is at the last position and posture of the ultrasound probe 1 in a case of acquiring the three-dimensional ultrasound image data of which the position of the occupied region R1 is estimated by the position estimation unit 27, or is at the position and the posture of the ultrasound probe 1, which is closest to the current position of the ultrasound probe 1, in a case of acquiring the three-dimensional ultrasound image data of which the position of the occupied region R1 is estimated by the position estimation unit 27.

As a result, the user can acquire again the ultrasound image from the position and the posture of the ultrasound probe 1 in a case of acquiring the three-dimensional ultrasound image data of which the position of the occupied region R1 is estimated by the position estimation unit 27. Therefore, the position of the occupied region R1 estimated by the position estimation unit 27 can be reliably estimated, and the occupied region R1 can be accurately displayed by the emphasized display unit 53. The user can more reliably and comprehensively image the necessary region in the subject while checking the occupied region R1 displayed in an emphasized manner on the monitor 23 as described above.

Note that the notification unit 55 has, for example, an interval threshold value for an interval between the synthesized three-dimensional ultrasound image data adjacent to each other, and can determine that the three-dimensional ultrasound image data can be synthesized at the correct position in a case where the interval between the three-dimensional ultrasound image data is equal to or less than the interval threshold value. In addition, for example, in a case where the interval between the synthesized three-dimensional ultrasound image data adjacent to each other is greater than the interval threshold value, the notification unit 55 can determine that the three-dimensional ultrasound image data is not synthesized at the correct position.

In addition, the notification unit 55 can also determine whether or not the three-dimensional ultrasound image data can be synthesized at the correct position on the basis of a similarity degree of a boundary portion in the two pieces of three-dimensional ultrasound image data which are adjacent to each other and are to be synthesized with each other. The notification unit 55 has, for example, a similarity degree threshold value for the similarity degree of the boundary portion in the two pieces of the three-dimensional ultrasound image data, and calculates the similarity degree of the boundary portion in the two pieces of the three-dimensional ultrasound image data. The notification unit 55 can determine that the three-dimensional ultrasound image data can be synthesized at the correct position in a case where the calculated similarity degree is equal to or greater than the similarity degree threshold value, and can determine that the three-dimensional ultrasound image data cannot be synthesized at the correct position in a case where the calculated similarity degree is less than the similarity degree threshold value.

In addition, the notification unit 55 has a smoothness threshold value for the smoothness of the three-dimensional structure in the synthesized three-dimensional ultrasound image data, and calculates the smoothness of the three-dimensional structure in the synthesized three-dimensional ultrasound image data. The notification unit 55 can determine that the three-dimensional ultrasound image data can be synthesized at the correct position in a case where the calculated smoothness is equal to or greater than the smoothness threshold value, and can determine that the three-dimensional ultrasound image data cannot be synthesized at the correct position in a case where the calculated smoothness is less than the smoothness threshold value. Here, the smoothness of the three-dimensional structure is an index value having a high value as the surface of the three-dimensional structure has a smooth shape and having a low value as the surface of the three-dimensional structure has an irregular shape.

In addition, the notification unit 55 can store an ideal three-dimensional structure in the subject in advance, calculate a similarity degree between the three-dimensional structure in the synthesized three-dimensional ultrasound image data and the ideal three-dimensional structure, and determine whether or not the three-dimensional ultrasound image data can be synthesized at the correct position by determining whether or not the similarity degree is equal to or greater than the predetermined threshold value.

In addition, the description has been made in which the notification unit 55 displays a message or the like on the monitor 23 to notify the user, but the notification method by the notification unit 55 is not particularly limited thereto. For example, in a case where the ultrasound diagnostic apparatus comprises a speaker (not illustrated), the notification unit 55 can notify the user by sound via the speaker. In addition, for example, in a case where the ultrasound probe 1 comprises a vibration device (not illustrated), the notification unit 55 can notify the user by vibrating the ultrasound probe 1 using the vibration device. Here, the vibration device is a device that includes a so-called vibration motor or the like and vibrates the ultrasound probe 1. In addition, for example, in a case where the ultrasound diagnostic apparatus comprises a lamp (not illustrated), the notification unit 55 can notify the user by turning on the lamp. As described above, the notification unit 55 can notify the user by appealing to the user's five senses by various methods.

### Sixth Embodiment

The user having a low skill level in the examination using the ultrasound diagnostic apparatus may not be able to ascertain which part of the subject the ultrasound image is acquired from, and may not be able to ascertain whether or not the intended part is imaged even though the acquired ultrasound image is checked. Therefore, the ultrasound diagnostic apparatus can present the ultrasound image to be observed in advance.

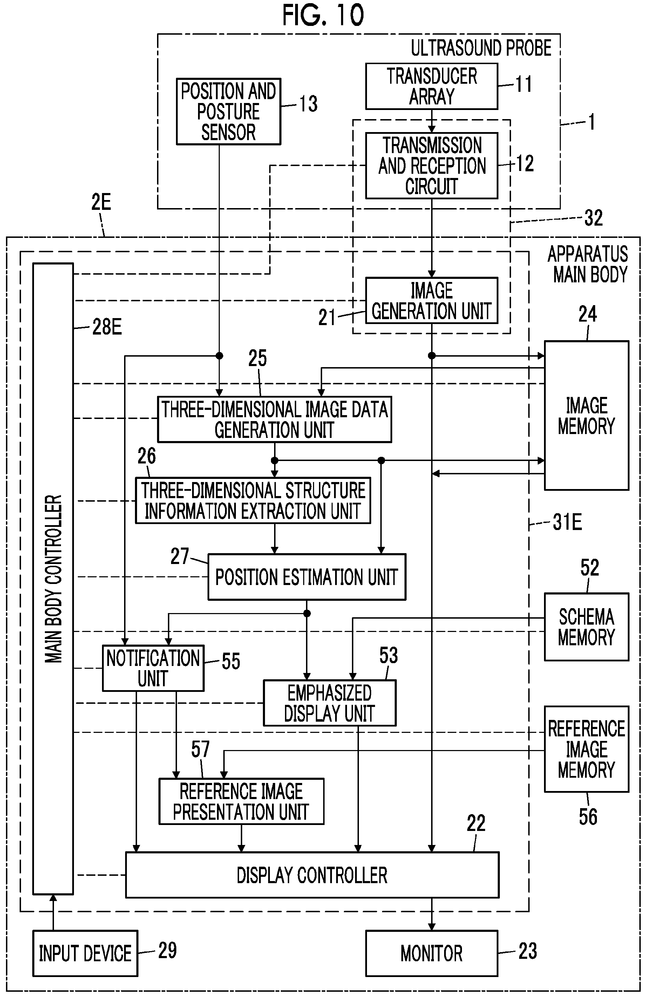

Fig. 10 is a block diagram illustrating a configuration of an ultrasound diagnostic apparatus according to a sixth embodiment. The ultrasound diagnostic apparatus of the sixth embodiment is obtained by including an apparatus main body 2E instead of the apparatus main body 2 in the ultrasound diagnostic apparatus of the first embodiment illustrated in Fig. 1. The apparatus main body 2E is obtained by further providing the schema memory 52, the emphasized display unit 53, the notification unit 55, a reference image memory 56, and a reference image presentation unit 57 to the apparatus main body 2 in the first embodiment, and including a main body controller 28E instead of the main body controller 28. Here, the schema memory 52 and the emphasized display unit 53 are the same as the schema memory 52 and the emphasized display unit 53 in the third to fifth embodiments, and the notification unit 55 is the same as the notification unit 55 in the fifth embodiment.

In the apparatus main body 2E, the reference image memory 56 is connected to the main body controller 28E. In addition, the reference image presentation unit 57 is connected to the notification unit 55 and the reference image memory 56. The reference image presentation unit 57 is connected to the display controller 22 and the main body controller 28E. In addition, a processor 31E for the apparatus main body 2E is configured by the image generation unit 21, the display controller 22, the three-dimensional image data generation unit 25, the three-dimensional structure information extraction unit 26, the position estimation unit 27, the main body controller 28E, the emphasized display unit 53, the notification unit 55, and the reference image presentation unit 57.

The reference image memory 56 is a memory that stores a plurality of reference ultrasound images to be observed in each part inside the subject in advance. As the plurality of reference ultrasound images, for example, a plurality of ultrasound images acquired in the past examination and stored in association with the position and posture information of the ultrasound probe 1, a plurality of ultrasound images acquired by another ultrasound diagnostic apparatus (not illustrated) and stored in association with the position and posture information of the ultrasound probe used in the other ultrasound diagnostic apparatus, and the like can be used. In addition, for example, recording media such as a flash memory, an HDD, an SSD, an FD, an MO disc, an MT, a RAM, a CD, a DVD, an SD card, and a USB memory can be used as the reference image memory 56.

The notification unit 55 specifies a region that is not displayed in an emphasized manner by the emphasized display unit 53 in the three-dimensional schema image A displayed on the monitor 23 and notifies the user of the specified region.

The reference image presentation unit 57 reads out the reference ultrasound image to be observed in the region specified by the notification unit 55, from the reference image memory 56, and displays the reference ultrasound image on the monitor 23. The reference image presentation unit 57 can extract at least one reference ultrasound image corresponding to the position of the region specified by the notification unit 55 with reference to the position and posture information of the ultrasound probe 1 corresponding to the plurality of reference ultrasound images stored in the reference image memory 56, and can present the at least one reference ultrasound image on the monitor 23.

Since the user captures the ultrasound image while checking the reference ultrasound image presented on the monitor 23, the user can sequentially capture the ultrasound images while easily ascertaining whether or not the intended part is imaged even in a case where the user's skill level is low. Accordingly, the user can more reliably and comprehensively image the necessary region in the subject.

### Explanation of References

1: ultrasound probe
2, 2A, 2B, 2C, 2D, 2E: apparatus main body
11: transducer array
12: transmission and reception circuit
13: position and posture sensor
21: image generation unit
22: display controller
23: monitor
24: image memory
25: three-dimensional image data generation unit
26: three-dimensional structure information extraction unit
27: position estimation unit
28, 28A, 28B, 28C, 28D, 28E: main body controller
29: input device
31, 31A, 31B, 31C, 31D, 31E: processor
32: image acquisition unit
41: pulser
42: amplification unit
43: AD conversion unit
44: beam former
45: signal processing unit
46: DSC
47: image processing unit
51: reconstruction unit
52: schema memory
53: emphasized display unit
54: data synthesis unit
55: notification unit
56: reference image memory
57: reference image presentation unit
A: three-dimensional schema image
B1: liver
B2: gallbladder
B3: stomach
B4: spleen
B5: pancreas
B6: duodenum
R1: occupied region

## Claims

1. An ultrasound diagnostic apparatus comprising:
an ultrasound probe (1);
a position and posture sensor (13) that acquires position and posture information of the ultrasound probe (1);
an image acquisition unit (32) that acquires an ultrasound image representing a tomogram of a subject by transmitting and receiving an ultrasound beam using the ultrasound probe (1);
a three-dimensional image data generation unit (25) that generates three-dimensional ultrasound image data of the subject on the basis of the position and posture information of the ultrasound probe (1) acquired by the position and posture sensor (13) and the ultrasound image acquired by the image acquisition unit (32);
a three-dimensional structure information extraction unit (26) that extracts three-dimensional structure information regarding a three-dimensional structure included in the three-dimensional ultrasound image data from the three-dimensional ultrasound image data generated by the three-dimensional image data generation unit (25); and
a position estimation unit (27) that has a position estimation model trained in a position of the three-dimensional structure in the three-dimensional image data obtained by imaging the three-dimensional structure, and estimates a position of a region occupied by the three-dimensional ultrasound image data of the subject by the position estimation model on the basis of the three-dimensional structure information extracted by the three-dimensional structure information extraction unit (26);
**characterized by** a reconstruction unit (51) that extracts a two-dimensional tomographic image from the three-dimensional ultrasound image data generated by the three-dimensional image data generation unit (25), and reconstructs the three-dimensional ultrasound image data of the subject on the basis of the extracted tomographic image and the position and posture information of the ultrasound probe (1) acquired by the position and posture sensor (13),
wherein the three-dimensional structure information extraction unit (26) extracts the three-dimensional structure information from the three-dimensional ultrasound image data reconstructed by the reconstruction unit (51).

2. The ultrasound diagnostic apparatus according to claim 1, further comprising:
a monitor (23) that displays a three-dimensional schema image; and
an emphasized display unit (53) that displays, in an emphasized manner in the three-dimensional schema image, the occupied region of the three-dimensional ultrasound image data of which the position is estimated by the position estimation unit (27).

3. The ultrasound diagnostic apparatus according to claim 2, further comprising:
a data synthesis unit (54) that sequentially synthesizes the three-dimensional ultrasound image data generated by the three-dimensional image data generation unit based on the three-dimensional structure included in the three-dimensional ultrasound image data of which the position of the occupied region is estimated by the position estimation unit (27).

4. The ultrasound diagnostic apparatus according to claim 2 or 3, further comprising:
a notification unit (55) that, in a case where the position of the occupied region of the three-dimensional ultrasound image data is not able to be estimated by the position estimation unit (27), prompts a user to move the ultrasound probe (1) such that the ultrasound probe (1) is at a position and a posture of the ultrasound probe (1) in a case of acquiring the three-dimensional ultrasound image data of which the position of the occupied region has already been estimated by the position estimation unit (27), on the basis of the position and posture information of the ultrasound probe (1) acquired by the position and posture sensor (13).

5. The ultrasound diagnostic apparatus according to any one of claims 2 to 4, further comprising:
a notification unit (55) that specifies a region that is not displayed in an emphasized manner by the emphasized display unit (53) and notifies a user of the specified region.

6. The ultrasound diagnostic apparatus according to claim 5, further comprising:
a reference image presentation unit (57) that presents a reference ultrasound image to be observed in the region specified by the notification unit (55).

7. The ultrasound diagnostic apparatus according to any one of claims 1 to 6,
wherein the three-dimensional structure information includes a shape pattern of the three-dimensional structure.

8. The ultrasound diagnostic apparatus according to claim 7,
wherein the three-dimensional structure information includes a brightness pattern of the three-dimensional structure.

9. The ultrasound diagnostic apparatus according to any one of claims 1 to 8,
wherein the position and posture sensor (13) includes an inertial sensor, a magnetic sensor, or an optical sensor.

10. A control method of an ultrasound diagnostic apparatus, the control method comprising:
acquiring position and posture information of an ultrasound probe (1);
acquiring an ultrasound image representing a tomogram of a subject by transmitting and receiving an ultrasound beam using the ultrasound probe (1);
generating three-dimensional ultrasound image data of the subject on the basis of the acquired position and posture information of the ultrasound probe (1) and the acquired ultrasound image;
extracting three-dimensional structure information regarding a three-dimensional structure included in the three-dimensional ultrasound image data from the generated three-dimensional ultrasound image data; and
estimating a position of a region occupied by the three-dimensional ultrasound image data of the subject by inputting the extracted three-dimensional structure information to a position estimation model trained in a position of the three-dimensional structure in the three-dimensional image data obtained by imaging the three-dimensional structure
**characterized in that** the method further comprises extracting a two-dimensional tomographic image from the generated three-dimensional ultrasound image data, and reconstructing the three-dimensional ultrasound image data of the subject on the basis of the extracted tomographic image and the position and posture information of the ultrasound probe (1); and
extracting the three-dimensional structure information from the reconstructed three-dimensional ultrasound image data.

## Patentansprüche

1. Ultraschalldiagnosevorrichtung, umfassend:
eine Ultraschallsonde (1);
einen Positions- und Stellungssensor (13), der Positions- und Stellungsinformationen der Ultraschallsonde (1) erfasst;
eine Bilderfassungseinheit (32), die ein Ultraschallbild, das ein Tomogramm einer Untersuchungsperson darstellt, durch Übertragen und Empfangen eines Ultraschallstrahls unter Verwendung der Ultraschallsonde (1) erfasst;
eine Erzeugungseinheit (25) für dreidimensionale Bilddaten, die dreidimensionale Ultraschallbilddaten der Untersuchungsperson auf der Grundlage der Positions- und
Stellungsinformationen der Ultraschallsonde (1), die von dem Positions- und
Stellungssensor (13) erfasst worden sind, und des Ultraschallbildes, das von der Bilderfassungseinheit (32) erfasst worden ist, erzeugt;
eine Extraktionseinheit (26) für dreidimensionale Strukturinformationen, die dreidimensionale Strukturinformationen in Bezug auf eine dreidimensionale Struktur,
die in den dreidimensionalen Ultraschallbilddaten enthalten ist, aus den dreidimensionalen Ultraschallbilddaten, die von der Erzeugungseinheit (25) für dreidimensionale Bilddaten erzeugt worden sind, extrahiert; und
eine Positionsschätzungseinheit (27), die ein Positionsschätzmodell, das in einer Position der dreidimensionalen Struktur in den dreidimensionalen Bilddaten, die durch Abbilden der dreidimensionalen Struktur erhalten worden sind, trainiert worden ist, aufweist und eine Position eines Bereichs, der von den dreidimensionalen Ultraschallbilddaten der Untersuchungsperson eingenommen wird, durch das Positionsschätzmodell auf der Grundlage der dreidimensionalen Strukturinformationen, die von der Extraktionseinheit (26) für dreidimensionale Strukturinformationen extrahiert worden sind, schätzt;
**gekennzeichnet durch** eine Rekonstruktionseinheit (51), die ein zweidimensionales Tomographiebild aus den dreidimensionalen Ultraschallbilddaten, die von der Erzeugungseinheit (25) für dreidimensionale Bilddaten erzeugt worden sind, extrahiert und die dreidimensionalen Ultraschallbilddaten der Untersuchungsperson auf der Grundlage des extrahierten Tomographiebildes und der Positions- und
Stellungsinformationen der Ultraschallsonde (1), die von dem Positions- und Stellungssensor (13) erfasst worden sind, rekonstruiert,
wobei die Extraktionseinheit (26) für dreidimensionale Strukturinformationen die dreidimensionalen Strukturinformationen aus den dreidimensionalen Ultraschallbilddaten, die von der Rekonstruktionseinheit (51) rekonstruiert worden sind, extrahiert.

2. Ultraschalldiagnosevorrichtung nach Anspruch 1, ferner umfassend:
einen Monitor (23), der ein dreidimensionales Schemabild anzeigt; und
eine Hervorhebungsanzeigeeinheit (53), die in dem dreidimensionalen Schemabild den eingenommenen Bereich der dreidimensionalen Ultraschallbilddaten, deren Position durch die Positionsschätzungseinheit (27) geschätzt wird, auf hervorgehobene Weise anzeigt.

3. Ultraschalldiagnosevorrichtung nach Anspruch 2, ferner umfassend:
eine Datensyntheseeinheit (54), die die dreidimensionalen Ultraschallbilddaten, die von der Erzeugungseinheit für dreidimensionale Bilddaten erzeugt worden sind,
sequentiell auf der Grundlage der dreidimensionalen Struktur, die in den dreidimensionalen Ultraschallbilddaten enthalten ist, deren Position des eingenommenen Bereichs durch die Positionsschätzungseinheit (27) geschätzt wird, synthetisiert.

4. Ultraschalldiagnosevorrichtung nach Anspruch 2 oder 3, ferner umfassend:
eine Benachrichtigungseinheit (55), die in einem Fall, in dem die Position des eingenommenen Bereichs der dreidimensionalen Ultraschallbilddaten nicht durch die Positionsschätzungseinheit (27) geschätzt werden kann, einen Benutzer auffordert, die Ultraschallsonde (1) so zu bewegen, dass sich die Ultraschallsonde (1) an einer Position und einer Stellung der Ultraschallsonde (1) in einem Fall des Erfassens der dreidimensionalen Ultraschallbilddaten, deren Position des eingenommenen Bereichs bereits durch die Positionsschätzungseinheit (27) geschätzt worden ist, auf der Grundlage der Positions- und Stellungsinformationen der Ultraschallsonde (1), die von dem Positions- und Stellungssensor (13) erfasst worden sind, befindet.

5. Ultraschalldiagnosevorrichtung nach einem der Ansprüche 2 bis 4, ferner umfassend:
eine Benachrichtigungseinheit (55), die einen Bereich, der von der Hervorhebungsanzeigeeinheit (53) nicht auf hervorgehobene Weise angezeigt wird, spezifiziert und einen Benutzer über den spezifizierten Bereich benachrichtigt.

6. Ultraschalldiagnosevorrichtung nach Anspruch 5, ferner umfassend:
eine Referenzbild-Präsentationseinheit (57), die ein Referenz-Ultraschallbild, das in dem von der Benachrichtigungseinheit (55) spezifizierten Bereich zu beobachten ist, präsentiert.

7. Ultraschalldiagnosevorrichtung nach einem der Ansprüche 1 bis 6,
wobei die dreidimensionalen Strukturinformationen ein Formmuster der dreidimensionalen Struktur enthalten.

8. Ultraschalldiagnosevorrichtung nach Anspruch 7,
wobei die dreidimensionalen Strukturinformationen ein Helligkeitsmuster der dreidimensionalen Struktur enthalten.

9. Ultraschalldiagnosevorrichtung nach einem der Ansprüche 1 bis 8,
wobei der Positions- und Stellungssensor (13) einen Trägheitssensor, einen Magnetsensor oder einen optischen Sensor enthält.

10. Steuerverfahren einer Ultraschalldiagnosevorrichtung, wobei das Steuerverfahren umfasst:
Erfassen von Positions- und Stellungsinformationen einer Ultraschallsonde (1);
Erfassen eines Ultraschallbildes, das ein Tomogramm einer Untersuchungsperson darstellt, durch Übertragen und Empfangen eines Ultraschallstrahls unter Verwendung der Ultraschallsonde (1);
Erzeugen von dreidimensionalen Ultraschallbilddaten der Untersuchungsperson auf der Grundlage der erfassten Positions- und Stellungsinformationen der Ultraschallsonde (1) und des erfassten Ultraschallbildes;
Extrahieren von dreidimensionalen Strukturinformationen in Bezug auf eine dreidimensionale Struktur, die in den dreidimensionalen Ultraschallbilddaten enthalten ist, aus den erzeugten dreidimensionalen Ultraschallbilddaten; und
Schätzen einer Position eines Bereichs, der von den dreidimensionalen Ultraschallbilddaten der Untersuchungsperson eingenommen wird, durch Eingeben der extrahierten dreidimensionalen Strukturinformationen in ein Positionsschätzmodell, das in einer Position der dreidimensionalen Struktur in den dreidimensionalen Bilddaten, die durch Abbilden der dreidimensionalen Struktur erhalten werden, trainiert worden ist;
**dadurch gekennzeichnet, dass** das Verfahren ferner Extrahieren eines zweidimensionalen Tomographiebildes aus den erzeugten dreidimensionalen Ultraschallbilddaten und Rekonstruieren der dreidimensionalen Ultraschallbilddaten der Untersuchungsperson auf der Grundlage des extrahierten Tomographiebildes und der Positions- und Stellungsinformationen der Ultraschallsonde (1) umfasst; und
Extrahieren der dreidimensionalen Strukturinformationen aus den rekonstruierten dreidimensionalen Ultraschallbilddaten.

## Revendications

1. Appareil de diagnostic par ultrasons comprenant :
une sonde ultrasonore (1) ;
un capteur de position et de posture (13) qui acquiert des informations de position et de posture de la sonde ultrasonore (1) ;
une unité d'acquisition d'images (32) qui acquiert une image ultrasonore représentant un tomogramme d'un sujet en transmettant et en recevant un faisceau ultrasonore en utilisant la sonde ultrasonore (1) ;
une unité de génération de données d'images tridimensionnelles (25) qui génère des données d'images ultrasonores tridimensionnelles du sujet sur la base des informations de position et de posture de la sonde ultrasonore (1) acquises par le capteur de position et de posture (13) et de l'image ultrasonore acquise par l'unité d'acquisition d'images (32) ;
une unité d'extraction d'informations de structure tridimensionnelle (26) qui extrait des informations de structure tridimensionnelle concernant une structure tridimensionnelle incluse dans les données d'images ultrasonores tridimensionnelles à partir des données d'images ultrasonores tridimensionnelles générées par l'unité de génération de données d'images tridimensionnelles (25) ; et
une unité d'estimation de position (27) qui a un modèle d'estimation de position entraîné dans une position de la structure tridimensionnelle dans les données d'images tridimensionnelles obtenues par imagerie de la structure tridimensionnelle, et estime une position d'une région occupée par les données d'images ultrasonores tridimensionnelles du sujet par le modèle d'estimation de position sur la base des informations de structure tridimensionnelle extraites par l'unité d'extraction d'informations de structure tridimensionnelle (26) ;
**caractérisé par** une unité de reconstruction (51) qui extrait une image tomographique bidimensionnelle des données d'images ultrasonores tridimensionnelles générées par l'unité de génération de données d'images tridimensionnelles (25), et reconstruit les données d'images ultrasonores tridimensionnelles du sujet sur la base de l'image tomographique extraite et des informations de position et de posture de la sonde ultrasonore (1) acquises par le capteur de position et de posture (13),
dans lequel l'unité d'extraction d'informations de structure tridimensionnelle (26) extrait les informations de structure tridimensionnelle des données d'images ultrasonores tridimensionnelles reconstruites par l'unité de reconstruction (51).

2. Appareil de diagnostic par ultrasons selon la revendication 1, comprenant en outre :
un moniteur (23) qui affiche une image schématique tridimensionnelle ; et
une unité d'affichage mise en évidence (53) qui affiche, de manière mise en évidence dans l'image schématique tridimensionnelle, la région occupée des données d'images ultrasonores tridimensionnelles dont la position est estimée par l'unité d'estimation de position (27).

3. Appareil de diagnostic par ultrasons selon la revendication 2, comprenant en outre :
une unité de synthèse de données (54) qui synthétise séquentiellement les données d'images ultrasonores tridimensionnelles générées par l'unité de génération de données d'images tridimensionnelles sur la base de la structure tridimensionnelle incluse dans les données d'images ultrasonores tridimensionnelles dont la position de la région occupée est estimée par l'unité d'estimation de position (27).

4. Appareil de diagnostic par ultrasons selon la revendication 2 ou la revendication 3, comprenant en outre :
une unité de notification (55) qui, dans un cas où la position de la région occupée des données d'images ultrasonores tridimensionnelles ne peut pas être estimée par l'unité d'estimation de position (27), invite un utilisateur à déplacer la sonde ultrasonore (1) de sorte que la sonde ultrasonore (1) soit à une position et une posture de la sonde ultrasonore (1) dans un cas d'acquisition des données d'images ultrasonores tridimensionnelles dont la position de la région occupée a déjà été estimée par l'unité d'estimation de position (27), sur la base des informations de position et de posture de la sonde ultrasonore (1) acquises par le capteur de position et de posture (13).

5. Appareil de diagnostic par ultrasons selon l'une quelconque des revendications 2 à 4, comprenant en outre :
une unité de notification (55) qui spécifie une région qui n'est pas affichée de manière mise en évidence par l'unité d'affichage en évidence (53) et notifie un utilisateur de la région spécifiée.

6. Appareil de diagnostic par ultrasons selon la revendication 5, comprenant en outre :
une unité de présentation d'images de référence (57) qui présente une image ultrasonore de référence à observer dans la région spécifiée par l'unité de notification (55).

7. Appareil de diagnostic par ultrasons selon l'une quelconque des revendications 1 à 6, dans lequel les informations de structure tridimensionnelle incluent un motif de forme de la structure tridimensionnelle.

8. Appareil de diagnostic par ultrasons selon la revendication 7,
dans lequel les informations de structure tridimensionnelle incluent un motif de luminosité de la structure tridimensionnelle.

9. Appareil de diagnostic par ultrasons selon l'une quelconque des revendications 1 à 8, dans lequel le capteur de position et de posture (13) inclut un capteur inertiel, un capteur magnétique ou un capteur optique.

10. Procédé de contrôle d'un appareil de diagnostic par ultrasons, le procédé de contrôle comprenant :
acquérir des informations de position et de posture d'une sonde ultrasonore (1) ;
acquérir une image ultrasonore représentant un tomogramme d'un sujet en transmettant et en recevant un faisceau ultrasonore en utilisant la sonde ultrasonore (1) ;
générer des données d'images ultrasonores tridimensionnelles du sujet sur la base des informations de position et de posture de la sonde ultrasonore (1) acquises et de l'image ultrasonore acquise ;
extraire des informations de structure tridimensionnelle concernant une structure tridimensionnelle incluse dans les données d'images ultrasonores tridimensionnelles à partir des données d'images ultrasonores tridimensionnelles générées ; et
estimer une position d'une région occupée par les données d'images ultrasonores tridimensionnelles du sujet en introduisant les informations de structure tridimensionnelle extraites dans un modèle d'estimation de position entraîné dans une position de la structure tridimensionnelle dans les données d'images tridimensionnelles obtenues par imagerie de la structure tridimensionnelle ;
**caractérisé en ce que** le procédé comprend en outre extraire une image tomographique bidimensionnelle à partir des données d'images ultrasonores tridimensionnelles générées, et reconstruire les données d'images ultrasonores tridimensionnelles du sujet sur la base de l'image tomographique extraite et des informations de position et de posture de la sonde ultrasonore (1) ; et
extraire les informations de structure tridimensionnelle à partir des données d'images ultrasonores tridimensionnelles reconstruites.
